# EUROPEAN PATENT APPLICATION

(11) **EP 0 695 542 A1**
(43) Date of publication of application: **07.02.1996**
(21) Application number: 94202252.6
(22) Date of filing: 03.08.1994
(51) Int. Cl.: A61F 13/15, A61F 13/56

(54) **Absorbent article having undergarment side wrapping elements**

(71) Applicant: THE PROCTER & GAMBLE COMPANY, Cincinnati, Ohio 45202 (US)
(72) Inventor: Coles, Peter, D-65779 Kelkheim (DE); Ramos Blanco, Agustin, D-61440 Oberursel (DE)
(74) Representative: Bottema, Johan Jan

(57) **Abstract**

The present invention relates to absorbent articles (20) such as sanitary napkins, panty liners, and incontinence pads that have side wrapping elements (50) for folding around the side edges of the wearer's undergarment on each lateral side of the article. The components are designed to automatically wrap the sides of a wearer's undergarments when the undergarments are pulled up by having zones of extensibility (56). Importantly the zones of extensibility are spaced at an optimum lateral distance for improved comfort independent of the wearer's type of undergarments.

## Description

### FIELD OF THE INVENTION

The present invention relates to absorbent articles such as sanitary napkins, panty liners, and incontinence pads that have side wrapping elements for folding around the side edges of the wearer's undergarment on each lateral side of the article. The components are designed to automatically wrap the sides of a wearer's undergarments when the undergarments are pulled up by having zones of extensibility. Importantly the zones of extensibility are spaced at an optimum lateral distance for improved comfort independent of the wearer's type of undergarment.

### BACKGROUND OF THE INVENTION

Absorbent articles such as sanitary napkins, pantiliners, and incontinence pads are devices that are typically worn in the crotch region of an undergarment. These devices are designed to absorb and retain liquid and other discharges from the human body and to prevent body and clothing soiling. Sanitary napkins are a type of absorbent article worn by women in a pair of panties that is normally positioned between the wearer's legs, adjacent to the perineal area of the body. Sanitary napkins in particular with side wrapping elements, often also referred to as side flaps or wings, are disclosed in the literature and are available in the marketplace.

Generally when sanitary napkins are provided with side flaps, the side flaps extend laterally from a central absorbent means and are intended to be folded around the edges of the wearer's panties in the crotch region. Commonly, the side flaps are provided with an attachment means for either affixing them to the underside of the wearer's panties or to the opposing side flap. The side flaps are particularly effective for preventing exudates from soiling the edges of the wearer's panties.

Sanitary napkins having wings or side flaps of various types are disclosed in U.S. Patent 4,687,478, entitled "Shaped Sanitary Napkin With Flaps", U.S. Patent 4,608,047, entitled "Sanitary Napkin Attachment Means", U.S. Patent 4,589,876, entitled "Sanitary Napkin" and its Reexamination Patent No. B1 4,589,876, U.S. Patent 4,285,343, entitled "Sanitary Napkin". Sanitary napkins having wings are commonly viewed as providing good protection against soiling.

However, some women find applying sanitary napkins having side flaps to be inconvenient for various reasons. For instance, some women find it to be difficult to attach the side flaps to the underside of the crotch of their panties. This can be due to factors such as difficulties in folding the side flaps propperty into place and to stick them to the sanitary napkin. As a result, some women still prefer a sanitary napkin without side flaps. In addition, some women who generally prefer a sanitary napkin with side flaps, occasionally (such as during periods of light flow) prefer a sanitary napkin without. Therefore, there is a need for a sanitary napkin which provides an alternative to sanitary napkins having conventional side flaps while still providing a similar protection.

Several variations of sanitary napkins having conventional side flaps have been suggested. For example, U.S. Patent 4,911,701 discloses a sanitary napkin having elastic strands for providing a greater convex shape to the body-facing portion of the central absorbent and for enabling adhesive-free placement of the side flaps into the panties. U.S. Patent 4,940,462 discloses a sanitary napkin with longitudinally expandable flaps. The flaps are designed to fold over the exterior of the wearer's panty and then to expand to conform with the contour of the panties. Further improvements have been disclosed in US application 08/124180 of Sept. 17, 1993 and 08/277733 of July 20, 1994.
It now has been found that the folding of the side flaps around the undergarments by means of extensibile portions as suggested by the last cited prior art reference (US application 08/277733) can further be improved for comfort performance and comfort perception. Undergarments having a paticularly high leg cut as discussed in detail below have only recently become popular to a general public but are already used by a considerable fraction of women particularly in Europe. Women wearing these undergarments and using sanitary napkins with side flaps having extensible portions can experience excess material of the extensible portions being bunched together inside the undergarment or flaring outside the elastics of the undergarments. For these women this can cause a perceived lack of comfort impression and can possibly even cause physical iritations. Therefore it is important for high leg cut undergarments that the extensible portions in the side flaps in laterally opposing sides cannot bunch up inside the undergarment during use. At the same time the absorbent articles should not deteriorate as regards to any of the performance aspect previously satisfied and which are relevant for wearers including those who prefer low cut undergarments.

Thus, a need exists for an absorbent article, such as a sanitary napkin, that is provided with an alternative to conventional side flaps which provides all the performance aspects (soiling protection, ease of handling ans so forth) of prior art side flaps having extensible portions and which can conveniently and efficiently solve the problems outlined above.

It is another object of the present invention to provide an absorbent article, such as a sanitary napkin, that is able to wrap around the sides of the wearer's panties and stay in place without providing the side flaps with panty fasteners thereon, and without the need to attach separate elastic strands to the sanitary napkin.

These and other objects of the present invention will be more readily apparent when considered in reference to the following description and when taken in conjunction with the accompanying drawings.

### SUMMARY OF THE INVENTION

The present invention provides an absorbent article, such as a sanitary napkin. The sanitary napkin of the present invention has a pair of side wrapping elements (or "undergarment covering components") that provide coverage to the wearer's panties to reduce side soiling (i.e., staining of the edges of the panty crotch) without the use of conventional flaps.

The sanitary napkin comprises a main body portion comprising a liquid pervious topsheet, a liquid impervious backsheet joined to the topsheet, and an absorbent core positioned between the topsheet and the backsheet. The side wrapping elements are either integrally formed with the main body portion or joined to the garment-facing side of the main body portion inboard of the longitudinal side edges thereof. The side wrapping elements comprise a pair of flexible elements that extend beyond the crotch edge portions of the wearer's undergarment.

The side wrapping elements are each provided with at least one zone of extensibility, preferably two spaced apart zones of extensibility, which are symetrically placed in respect to the longitudinal centerline of the absorbent article. The zones of extensibility are regions of the side wrapping elements that have a greater range of extension than the adjacent regions of the side wrapping elements. The zones of extensibility have laterally most inward points which are located closest to the longitudinal centerline. These laterally most inward points have a distance to the longitudinal centerline of at least 40 mm, preferably 40 mm to 60 mm.

While not wishing to be bound by theory it is believed that this placement of the zones of extensibility (spaced away from the longitudinal centerline of the absorbent article) ensures the desired comfort improvement according to the present invention for undergarments having a high leg cut design. In this undergarment design the required amount of extensibility is not as large as in undergarments having a low leg cut design. Therefore if zones of extensibility are designed to accomodate the maximum extensibility requirements they will have some excess material in high leg cut undergarments. This excess material can easily flare outside the elastics of the undergarment or bunch together and cause perceived and real irritations to the wearer.
In high leg cut undergarments the zones of extensibility according to the present invention will come to rest with their inner edges in proximity to the crotch edge portions of the wearer's undergarment, i.e. the zones will mainly be outbord of the undergarment crotch region, when first placing the article in the undergarment. Upon folding the side flaps downward, e.g. when lifting the undergarment into place, the zones of extensibility are placed below the undergarment from a wearer point of view. Thus the material in the zones of extensibility which is not stretched out and potentially bunches together will do so outside the undergarment, i.e. not in contact with the wearer's skin. It hence cannot cause real skin irritations. At the same time perceived irritation is prevented because when pulling the undergarment down (e.g. to check placement of the sanitary napkin) the wearer cannot see any bunched together materials in the zones of extensibility since they are hidden below the crotch portion of the undergarment.
For low leg cut undergarments the zones of extensibility according to the present invention will come to rest extending laterally over the crotch edge portion of the wearer's undergarment, i.e. the zones will essentially be centered around the undergarment crotch edges. For undergarments having a particularly wide crotch width the zones can even be mainly inboard of the undergarment crotch edge. Upon folding the side flaps downward, e.g. when lifting the undergarment into place, the zones of extensibility are placed inside the undergarment and around the undergarment crotch edge. However for these low leg cut undergarments and in this position the extensible zones are almost fully extended and hence no bunching or flaring out occur while the benefits of the side flaps with extensible zones are maintained.

If the side wrapping elements are formed integrally with the main body portion of the absorbent article, notch regions are formed extending around the intersection between the perimeter of the side wrapping elements and the longitudinal side edge of the main body portion where the side wrapping elements extend beyond the longitudinal side edge of the main body portion. For these integral side wrapping element designs it is preferable when at least one symetric pair of zones of extensibility extend into said notch regions, i.e. each extensible zone extends across the longitudinal side edge of the main body portion.

Alternatively for side wrapping elements which are provided as separate elements and which are attached to said garment-facing side (and which are unattached outward from where they are attached) the distance between the laterally most inward points of the zones of extensibility and the longitudinal centerline is preferably in the range of 40 mm to 50 mm.

Generally the zone of extensibility can be primarily extensible in the longitudinal direction or primarily in the transverse direction or in any direction falling therebetween. For integrally formed side wrapping elements the zone of extensibility is most preferrably extensible in a direction following as close as possible the adjacent outer perimeter of the absorbent article. Preferably the zones of extensibility are provided with corrugations having fold lines.

In a preferred embodiment of the present invention the side wrapping elements comprise at least one zone of extensibility on each side of the transverse centerline of the side wrapping elements and a stiffer, less extensible intermediate region along the transverse centerline of the side wrapping elements. Preferably the longitudinal distance between the zones of extensibility on opposite sides of the transverse centerline of the side wrapping elements is between 20 mm and 100 mm.

In this embodiment the side wrapping elements have improved resistance to bending, crumpling, and other types of transverse deformation than a similar side wrapping element would have if it were made of the same material and was provided with extensibility along its full length. The stiffer, less extensible intermediate region located along the transverse centerline of the side wrapping elements provides the side wrapping elements with the improved resistance to bending and crumpling. This helps the side wrapping elements to fold over the elasticated sides of the wearer's panties, and to not crumple when the wearer's thighs apply compressive forces on the absorbent article.
The fact that the side wrapping elements have crumpling resistance and zones of extensibility spaced according to the invention allows the side wrapping elements to automatically fold around the crotch edge portions of the wearer's undergarment toward the underside of the undergarment and to remain so folded over the crotch edge when the absorbent article is placed in an undergarment and the undergarment is pulled up adjacent the wearer's body. The zones of extensibility and stiffer intermediate region further improve the mechanism for controlling the manner and location of folding of the side wrapping elements.

The sanitary napkins of the present invention provide an alternative to conventional sanitary napkins having side flaps. In one embodiment the side wrapping elements require no action on the part of the wearer to fold them under her panties or to attach them to the panties. The side wrapping elements stay in place well enough to cover the sides edges of the wearer's panties without affixing them underneath the wearer's panties.

In an alternative embodiment, particularly for side wrapping elements extending far outside the main body portion, the sanitary napkin may be provided with a fastener, such as a pressure sensitive adhesive. The adhesive fastener may be provided on the garment-facing side of the main body portion and also extend onto the garment-facing side of the side wrapping elements. In this embodiment, particularly in narrow panty crotches, the side wrapping elements may fold around the side edge of the wearer's panty crotch so that portions of the side wrapping elements even overlap. This forms a novel structure that pinches the side edge of the panties between the folded portion of the side wrapping elements.

### BRIEF DESCRIPTION OF THE DRAWINGS

FIG. 1 is a top plan view of the sanitary napkin of the present invention.

FIG. 2 is a bottom plan view of the sanitary napkin shown in FIG. 1.

FIG. 3 shows a high leg cut undergarment when worn.

FIG. 4 shows the high leg cut undergarment of Fig. 3 cut apart at the hips and stretched out flat.

FIG. 5 shows a low leg cut undergarment when worn.

FIG. 6 shows the low leg cut undergarment of Fig. 5 cut apart at the hips and stretched out flat.

### DETAILED DESCRIPTION OF THE INVENTION

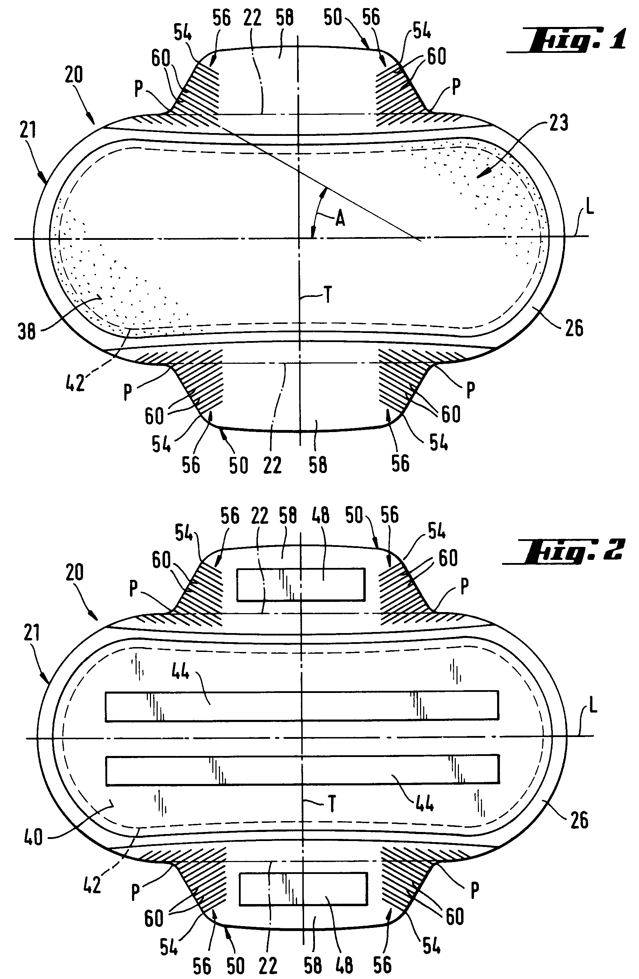

FIGS. 1-2 show one preferred embodiment of a disposable absorbent article of the present invention 20. The present invention relates to absorbent articles, such as sanitary napkins. More particularly, the present invention relates to sanitary napkins that have a main body portion 21 and a pair of side wrapping elements 50 that wrap the sides of the wearer's panties when the wearer places the sanitary napkin in her panties and pulls her panties up.

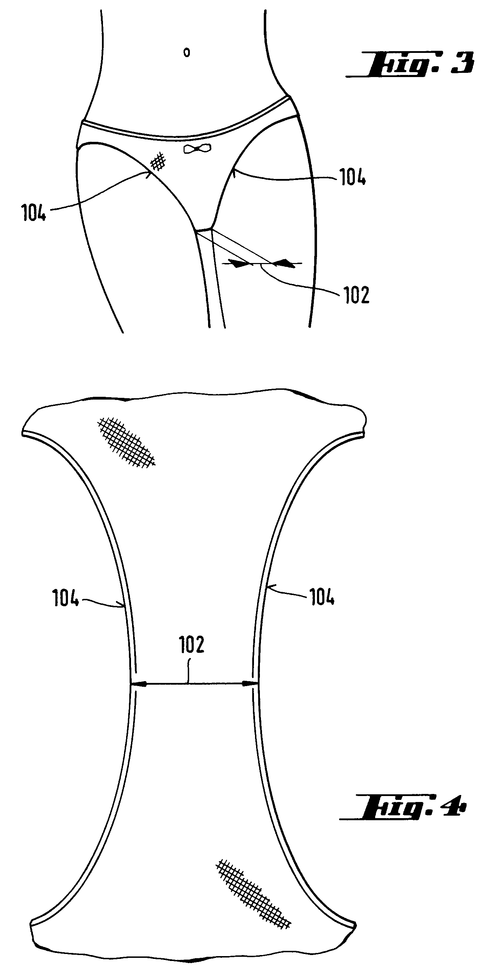

The sanitary napkin 20 has two surfaces, a liquid pervious body-contacting surface or "body surface" 23 and a liquid impervious garment surface. The sanitary napkin 20 is shown in FIG. 1 as viewed from its body surface 23. The body surface 23 is intended to be worn adjacent to the body of the wearer. The garment surface of the sanitary napkin 20 is shown in FIG. 2, it is on the opposite side and is intended to be placed adjacent to the wearer's undergarments when the sanitary napkin 20 is worn.

The sanitary napkin 20 has two centerlines, a longitudinal centerline L and a transverse centerline T. The term "longitudinal", as used herein, refers to a line, axis or direction in the plane of the sanitary napkin 20 that is generally aligned with (e.g., approximately parallel to) a vertical plane which bisects a standing wearer into left and right body halves when the sanitary napkin 20 is worn. The terms "transverse" or "lateral" used herein, are interchangeable, and refer to a line, axis or direction which lies within the plane of the sanitary napkin 20 that is generally perpendicular to the longitudinal direction.

FIG. 1 shows the main body portion 21 of the sanitary napkin 20. The main body portion (21) comprises the portion of the sanitary napkin without the side wrapping elements. The main body portion 21 has two spaced apart longitudinal edges 22. A detailed description of a sanitary napkin and its main body portion is contained in U.S. Patent 4,690,680.

The main body portion 21 of the sanitary napkin 20 can be of any thickness, including relatively thick, relatively thin, or even very thin. The embodiment of the sanitary napkin 20 shown in Figures 1 and 2 is intended to be an example of a relatively thin sanitary napkin, preferably an "ultra-thin" sanitary napkin. An "ultra-thin" sanitary napkin 20 as described in U.S. Patents 4,950,264 and 5,009,653 preferably has a caliper of less than about 3 millimeters.

FIG. 1 or 2 show the individual components of the main body portion 21 of the sanitary napkin 20 of the present invention. The main body portion 21 of the sanitary napkin generally comprises at least three primary components. These include a liquid pervious topsheet 38, a liquid impervious backsheet 40, and an absorbent core 42 positioned between the topsheet 38 and the backsheet 40. There are also occasions, however, when one or preferably more of these components, such as the backsheet and topsheet, can be replaced by a component that serves as part of the side wrapping elements 50 described below. The topsheet, the backsheet, and the absorbent core may be assembled in a variety of configurations known in the art (including so called "sandwich" products and "tube" products).

Several preferred sanitary napkin configurations are described generally in U.S. Patent 4,321,924, "Bordered Disposable Absorbent Article"; U.S. Patent 4,425,130, "Compound Sanitary Napkin"; U.S. Patent 4,950,264, "Thin, Flexible Sanitary Napkin"; U.S. Patent 5,308,346, "Elasticised Sanitary Napkin"; U.S. Patent Application Serial No. 08/096,121 entitled "Absorbent Articles Having Panty Covering Components That Naturally Wrap the Sides of Panties" filed July 22, 1993; and U.S. Patent Application Serial No. 08/124,180 entitled "Absorbent Articles Having Panty Covering Components Comprising Extensible Web Materials Which Exhibit Elastic-Like Behavior" filed September 17, 1993. The main body portion 21 of the sanitary napkin may also be comprised of one or more extensible components such as those sanitary napkins, and the like described in PCT Publication Nos. WO 93/01785 and 93/01786.

Figures 1-2 show a preferred embodiment of the sanitary napkin 20 assembled in a sandwich construction in which the topsheet 38 and the backsheet 40 have length and width dimensions generally larger than those of the absorbent core 42. The topsheet 38 and the backsheet 40 extend beyond the edges of the absorbent core 42 to thereby form portions of the periphery 26. The sanitary napkin 20 of the present invention comprises pair of side wrapping elements 50 that extend laterally outward beyond the longitudinal side edges 22 of the main body portion 21. In order to maintain the absorbent core in it's position and to provide structural integrety to the main body portion the topsheet is joyned to the backsheet in any usual fashion along the periphery. In figures 1 or 2 this is achieved by crimping the topsheet to the backsheet.

The side wrapping elements 50 can be of any suitable size and shape. The side wrapping elements 50 of the present invention may have any of the dimensions and characteristics set forth for the panty covering components in the aforementioned U.S. Patent Application Serial Nos. 08/096,121 and 08/124,180and in particular U.S. Patent Application Serial No. (case 5354).

The side wrapping elements 50 can, as shown in FIGS. 1-2, be intergral with the main body portion 21. Alternatively the side wrapping elements 50 can, however, be joined to the main body portion 21 in any suitable manner. The term "joined", as used herein, encompasses configurations in which an element is directly secured to another element by affixing the element directly to the other element; configurations in which the element is indirectly secured to the other element by affixing the element to intermediate member(s) which in turn are affixed to the other element; and configurations in which one element is integral with another element, i.e., one element is essentially part of the other element. Thus, the side wrapping elements 50 can be integral extensions of the topsheet 38 and backsheet 40 of the main body portion 21 as shown in Fig. 1 and 2. In other embodiments, instead of being integral, the side wrapping elements 50 can comprise a single or more separate component which are attached to the garment facing side of the absorbent article.

In the preferred embodiment shown in FIGS. 1-2, the side wrapping elements 50 each comprise a web of material having two zones of extensibility 56 therein. The extensibility of all the zones of extensibility 56 on the side wrapping elements 50 can be in the same direction. Alternatively, one or more of the zones of extensibility 56 may be extensible in a different direction. Also the extensibility in each zone 56 can vary if so desired.

The zones of extensibility 56 are preferably capable of extending between about 20% and about 90%, more preferably between about 50% and about 80%, and most preferably between about 70% and about 80% under the forces associated with wearing the sanitary napkin in a pair of panties. The zones of extensibility 56 are also preferably extensible without being elastic. Further, any inherent elasticity in the zones of extensibility 56 (that is, any tendency of the material comprising the zones of extensibility to return to its original dimension) is generally relatively low to non-existent.

FIG. 1-2 show preferred locations for the zones of extensibility 56 and the manner in which they are determined. In a particularly preferred embodiment according to the present invention the longitudinal center line of a sanitary napkin and the transverse center line of the sanitary napkin are simultaneously lines of symmetry for all components of the absorbent article. Therefore, the determination of the location of the zones of extensibility is only described with reference to one of the zones.
The most inward point of a zone of extensibility can be found by moving a theoretical line parallel to the longitudinal center line towards the zone of extensibility until the theoratical line first touches the zone of extensiblility. Then the distance between this theoretical line touching a zone of extensibility and the longitudinal center line is measured. For each zone of extensibility this distance has to be 40 mm or more, preferably from 40 mm to 60 mm.
In the preferred embodiment of figures 1 and 2 the longitudinal side edge 22 of the main body portion of the article has two points "P" of intersection with the perimeter of the side wrapping element on each side of the longitudinal centerline. Around each point P of intersection extends a notch region incorparating portions of the main body portion of the absorbent article as well as portions of the side wrapping elements. It is preferred that the zone of extensibility extend within the notch region such that at least part of the zones of extensibility extend from the side wrapping elements onto the main body portion in the area of the notch region but not into the region of the absorbent core 42. Determination of the location of the zone of extensibility as described above remains unchanged for these preferred embodiments.
The side wrapping elements 50, as shown in FIG. 1 and 2, preferably also have a trapezoidally-shaped intermediate region or zone 58 located between the zones of extensibility 56. The intermediate region 58 comprises a portion of the distal edges 54 of the side wrapping elements. This intermediate region 58 is stiffer (that is, more resistant to bending) than the zones of extensibility 56. The intermediate region 58 can also be less extensible than the portions of the side wrapping elements that comprise the zones of extensibility 56. The intermediate region 58 provides the side wrapping elements 50 with greater resistance to bending and crumpling so that the side wrapping elements will fold over the panty elastic, rather than crumple, when they are subject to compression by the wearer's thighs. The stiffer intermediate region 58 also helps to maintain panty elastic coverage when the wearer pulls her panties down to check the sanitary napkin for soiling, and then pulls her panties back up. The stiffer material ensures that the side wrapping elements do not fold upward over top of the main body portion, and stay on the same plane as the main body portion of the sanitary napkin, where flimsier material would be pushed upward during the motion associated with pulling the panties downward and might tend to fold upward over the topsheet when the panties are pulled back up. Upon pulling the panty and sanitary napkin back up, flimsier material may also not be caught by the wearer's thighs and go back into place. The side wrapping elements 50 should provide sufficient bending and crumpling resistance so that they will fold rather than crumple in use and automatically wrap the edges of the wearer's panties.

The side wrapping elements 50 can be made from any of the materials used in the construction of the main body portion 21 of the sanitary napkin. The side wrapping elements 50 in the embodiment shown in FIGS. 1-2 preferably comprise a laminate of two materials such as the topsheet and the backsheet material. Separately attached side wrapping elements can be made in the same fashion or from a different laminate, e.g. of a soft extensible coverstock material such as a longitudinally extensible spunbond nonwoven web or a soft extensible formed film, an optional extensible intermediate layer such as a three dimensional formed film, and an extensible liquid impervious backing such as a polyethylene film backsheet material.

Suitable material for the side wrapping elements include those useful to provide the topsheet, the backsheet and optionally intermediate layers. In this context suitable nonwoven webs include a product known as Spunbond PE, which can be obtained from Polybond, Incorporated of Waynesboro, VA and a product known as COROLIND PE, which can be obtained from Corovin GMBH of Germany.

As extensible intermediate layer or as topsheet material a variation of a three dimensional formed film known as DRI-WEAVE can be used. DRI-WEAVE is used as a topsheet on sanitary napkins manufactured by the Procter & Gamble Company, Cincinnati, Ohio under U.S. Patents 4,342,314 and 4,463,045 . The three dimensional film has an embossed thickness of between 0.38 mm to 0.89 mm and for use in the side wrapping elements can be apertured or not apertured all the way through as in the case of DRI-WEAVE topsheet material. For integral side wrapping elements the DRI-WEAVE topsheet can be formed so that the apertures are closed off on the longitudinal side of the film which forms the topsheet side of the side wrapping elements.

The polyethylene backsheet material is a film comprised of LLDPE that preferably has a caliper of between 0.001 mm-0.04 mm. A useful polyethylene film is known as #P18-1401 and is obtained from Clopay Corporation of Cincinnati, Ohio. This polyethylene film can be joyned to form the laminate of the side wrapping elements together with the nonwoven and/or the formed film by any suitable method such as by a spiral application of adhesives or slot coating, or it can be welded or extruded together.

The composite laminate preferably has a caliper of between 0.13 - 1.5 mm and is capable of extending between about 25-80% without tearing. The particular laminate is chosen to provide a soft body-contacting surface, good extensibility in the zones of extensibility when the zones of extensibility are formed therein, good resistance to folding and crumpling and a liquid impervious barrier.

The side wrapping elements 50 can be provided with zones of extensibility 56 in a non-limiting number of different manners. The side wrapping elements 50 may, for example, comprise a substantially inextensible material that is provided with extensible regions for the zones of extensibility. The extensible regions can be created in any suitable manner, including but not limited to mechanically straining, corrugating, "ring rolling", heating and deforming, subjecting portions of the side wrapping elements 50 to compression between mating plates, and the like.

In other embodiments, the different regions of the side wrapping elements can be provided by forming the side wrapping elements out of materials having different extensibilities. For example, the side wrapping elements 50 can be comprised of a laminate of an extensible material and an inextensible material. In such an embodiment, the inextensible material can be provided in the configuration of the side wrapping elements. The inextensible material can have holes cut out where the zones of extensibility are to be located. This inextensible material can then be laminated to the extensible material to form a side wrapping element with zones of extensibility where the holes were cut from the inextensible material.

The embodiment shown in FIGS. 1-2 has zones of extensibility 56 formed by ring rolling (or pre-corrugating) two regions of the side wrapping elements. Suitable methods for ring rolling are described in U.S. Patent 4,107,364, U.S. Patent 4,834,741, U.S. Patent 5,143,679 issued to, U.S. Patent 5,156,793 and U.S. Patent 5,167,897.

The side wrapping elements 50 can be provided with ring rolled corrugations having fold lines 60 that are angled away from the longitudinal centerline L. The fold lines 60 can form any angle with the longitudinal centerline. The fold lines 60 as shown in the embodiment in FIGS. 1 form an angle A with the longitudinal centerline L. The angle A in the shown embodiment is 30°.

The general location of the zones of extensibility 56 in the embodiment shown in FIGS. 1-2 is preferred for several reasons. The fact that the zones of extensibility 56 are spaced apart and separated by the stiffened intermediate region 58 provides improved resistance to bending and crumpling and more control over the manner of folding around the edges of the wearer's panties. The side wrapping elements 50 will typically fold in the zones of extensibility 56 and the intermediate region 58. This makes the side wrapping elements sturdier and capable of more reliable folding than if the side wrapping elements were made entirely extensible and/or were made of materials having the same stiffness over their entire area.

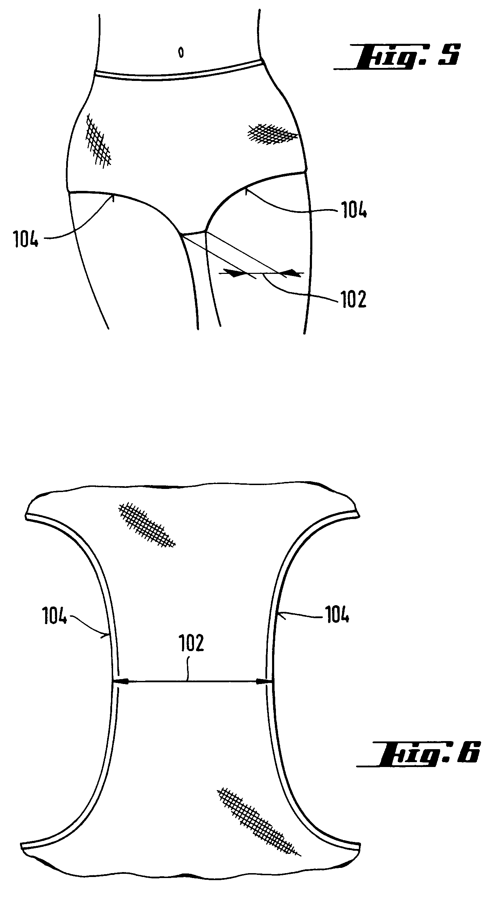

Another reason the zones of extensibility shown in FIGS. 1 or 2 are preferred is seen when considering the different dimensions found in undergarments. In this respect high leg cut undergarments and low leg cut undergarments can be distinguished. FIG. 3 shows an example of a high leg cut undergarment when worn and FIG. 4 shows the high leg cut undergarment of FIG. 3 when cut apart at the hips of the undergarment and stretched out flat. FIG. 5 shows a low leg cut undergarment when worn and FIG. 6 shows the same low leg cut undergarment of FIG. 5 when cut apart at the hips of the undergarment and stretched out flat. From both FIGS. 4 and 6 it can be seen that the crotch width portion 102 of the undergarment is one critical differentation between both styles of undergarments. While high leg cut undergarments have a crotch width of e.g. 74 mm, low leg cut undergarments having a crotch width 102 of e.g. 118 mm have been found. In addition the particular shape of bending along the central portion of the leg opening 104 of the undergarment is more gradual for low leg cut undergarments than for high leg cut undergarments. This effects the crotch width which has to be accommodated by the extensibility of the extensible zones of the side wrapping elements.

When placing a sanitary napkin in the crotch region of a high leg cut undergarment (which only recently have become increasingly popular with wearers of sanitary napkins) one can see that if the sanitary napkin has a too small distance between the zone of extensibility and the longitudinal centerline a substantial fraction of the zone of extensibility will be placed inside the undergarment from a wearer point of view. However, due to the shape of the high leg cut undergarment this extensible material will not be stretched to a full extend and therefore remain in a folded or corrogated state inside the undergarment.

This problem apparently cannot occur in the low leg cut undergarments since sanitary napkins having a small distance between the zone of extensibility and the longitudinal centerline will be stretched out flat around the side edge of the undergarment due to the particular shape of the low leg cut undergarment side edge and the wider crotch width.

Sanitary napkins according to the present invention solve the problem caused by the particular shape of the high leg cut undergarment while continuing to perform as desired for wearers of low leg cut undergarments. In particular for high leg cut undergarments the zone of extensibility will to a large extent be folded around the crotch edge of the undergarment such that any remaining corrugations or folds will come to lie outside of the undergarment from the wearers point of view. The increased stretching required by the low leg cut undergarments can, however, at the same time be supported by sanitary napkins according to the present invention. The place of extensibility in respect to the crotch edge of the undergarment is only relevant if a part of the extensible zone inside the undergarment remains unextended.

The garment surface of the sanitary napkin 20 may include, and preferably does include, fasteners for attaching the sanitary napkin to the wearer's undergarment. Figure 2 shows the central pad fastener 44 which is adapted to secure the portion of the sanitary napkin underlying the main body portion 21 to the crotch region of an undergarment. Any types of fasteners known in the art can be used. Fasteners comprising adhesives have been found to work well for this purpose, with pressure-sensitive adhesives being preferred. Before the sanitary napkin 20 is placed in use, if an adhesive fastener is used, the adhesive is typically covered with a removable cover strip or release liner in order to keep the adhesive from sticking to a surface other than the crotch portion of the panty prior to use. Suitable release liners are described in the U.S. Patent 4,917,697.

Fig. 2 shows a preferred arrangement which utilizes a pair of spaced apart longitudinally-oriented strips or zones of adhesive that are centered about the longitudinal centerline L. Further also centered around the longitudinal centerline L are tops, strips or zones 48 of adhesive on the side wrapping elements. They can be provided with the same or preferable with an alternative, stronger adhesive material and are also covered by release liners prior to use.

The sanitary napkin 20 of the present invention e.g. can be used by removing any release liner and thereafter placing the sanitary napkin 20 in a panty so that the adhesive 44 and other fastener 48 contacts the panty and maintains the sanitary napkin in position within the panty during use. The side wrapping elements 50 automatically wrap around the sides of the wearer's panties by the simple action of the wearer pulling up her panties or by being gently pushed around the undergarement croth edge by the wearer. The side wrapping elements 50 then assume an in-use position.

The operation of the side wrapping elements 50 is distinguishable in several aspects from that of conventional side flaps. First, placing a sanitary napkin having conventional flaps in a pair of panties and pulling up the panties will not consistently provide the automatic sustained wraparound feature of the present invention. There are several reasons for this. Conventional flaps are not provided with resistance to bending and crumpling so that they will tend to crumple in use, particularly when the wearer's thighs exert compressive forces on the flaps. Conventional flaps are also not provided with zones of extensibility, so they will generally not wrap around and conform to the panties. In those cases where conventional flaps and have zones of extensibility do wrap around the panties, they can have excess extensible flap material that can be conceived as being or actually be uncomfortable for the wearer. The side wrapping elements of the present invention, on the other hand wrap around the (elastic-containing) edges of the panties, but avoid the problems associated with excess flap material.

### Alternative Embodiments.

Numerous alternative embodiments of the present invention are possible. For example, the side wrapping elements are preferably mirror images of each other, and are symmetrical about the longitudinal centerline. However, it should be understood that the shape and location of the side wrapping elements described herein are those of a preferred embodiment, and other embodiments are also possible. For example, while the side wrapping elements 50 are shown as extending synetrical from each longitudinal edge of the main body portion, there may be one side wrapping element extending further than the other one. Further, the side wrapping elements 50 may be offset along the longitudinal centerline more towards one end edge of the main body portion than the other.

The terms "panty liner" refer to absorbent articles that are less bulky than sanitary napkins which are generally worn by women between their menstrual periods. Suitable absorbent articles in the form of pantiliners that can be provided with the side wrapping elements described herein are disclosed in U.S. Patent 4,738,676 entitled "Pantiliner".

The term "incontinence article" refers to pads, undergarments (pads held in place by a suspension system of same type, such as a belt, or the like), inserts for absorbent articles, capacity boosters for absorbent articles, briefs, bed pads, and the like, regardless of whether they are worn by adults, children or infants. Suitable incontinent articles that can be provided with the side wrapping elements described herein are disclosed in U.S. Patent 5,300,054 and U.S. Patent 5,304,161.

## Claims

1. An absorbent article for wearing in a wearer's undergarment that has a crotch region with a pair of side edges, said absorbent article having a longitudinal centerline extending parallel to a longitudinal direction , said absorbent article comprising:
a main body portion comprising an absorbent core, said main body portion having a body-facing side, a garment-facing side, and a pair of longitudinal side edges; and
a pair of side wrapping elements for folding around the side edges of the wearer'sundergarment, said side wrapping elements being joined to said main body portion and extending laterally outward beyond the longitudinal side edges of said main body portion; and
said side wrapping elements comprise at least one zone of extensibility each which are symetrically placed in respect to said longitudinal centerline and said zones of extensibility have laterally most inward points, said laterally most inward points being those points in said zones of extensibility that are located closest to said longitudinal centerline; and said absorbent article being characterised in that
said laterally most inward points have a distance to said longitudinal centerline of at least 40 mm.

2. An absorbent article according to Claim 1 wherein said laterally most inward points have a distance to said longitudinal centerline of 40 mm to 60 mm.

3. An absorbent article according to Claim 1 or 2 wherein said zones of extensibility are either primarily extensible in the longitudinal direction or primarily extensible in the transverse direction.

4. An absorbent article according to Claim 1 or 2 wherein said zones of extensibility are extensible in a direction between the longitudinal direction and the transverse direction.

5. An absorbent article according to any of the preceding claims wherein said side wrapping elements have a transverse centerline perpendicular to said longitudinal center line of the absorbent article and said side wrapping elements comprise at least two zones of extensibility on opposite sides of said transverse centerline and an intermediate region positioned between said zones of extensibility, said intermediate region containing said transverse centerline, preferably the longitudinal distance between said zones of extensibility on opposite sides of said transverse centerline is between 20 mm and 100 mm.

6. An absorbent article according to any of the preceding claims wherein at least one zone of extensibility is provided with corrugations having fold lines therein.

7. An absorbent article according to any of the preceding claims wherein said side wrapping elements are integral with said main body portion and form a notch region extending around the intersection between the perimeter of said side wrapping elements and the longitudinal side edge of said main body portion where said side wrapping elements extend beyond said longitudinal side edge of said main body portion.

8. An absorbent article according to claim 7 wherein at least one symetrical pair of zones of extensibility extend into said notch region.

9. An absorbent article according to any of the claims 1-6 wherein said side wrapping elements are provided as separate elements that are joined to the garment-facing side of said main body portion inward of the longitudinal side edges of said main body portion, and said side wrapping elements are unattached to said garment-facing side outward from where they are joyned.

10. An absorbent article according to claim 9 wherein said distance between said laterally most inward points of said zones of extensibility and said longitudinal centerline is from 40 mm to 50 mm.
